# EUROPEAN PATENT APPLICATION

(11) **EP 4 137 126 A1**
(43) Date of publication of application: **22.02.2023**
(21) Application number: 20931264.4
(22) Date of filing: 31.08.2020
(51) Int. Cl.: A61K 9/56, A61K 31/57, A61P 15/00

(54) **LONG-LASTING REABSORBABLE SUBCUTANEOUS IMPLANT WITH CONTROLLED RELEASE OF PRE-CONCENTRATED PHARMACOLOGICALLY ACTIVE SUBSTANCE IN POLYMER FOR THE TREATMENT OF ENDOMETRIOSIS**

(30) Priority: 17.04.2020 BR 102020007749
(71) Applicant: Luiz Peracchi, Edson, 81230-161 Curitiba (BR)
(72) Inventor: Luiz Peracchi, Edson, 81230-161 Curitiba (BR)
(74) Representative: De Pablos Riba, Juan Ramon
(86) International application number: PCT/BR2020/050344
(87) International publication number: WO 2021/207806

(57) **Abstract**

The present invention is aimed at the medical sector and comprises a biodegradable implant of gestrinone in a polymeric matrix. The implant is inserted subdermally and has a continuous release of the asset for an extended period of time. This release aims to guarantee an efficient, constant and prolonged serum level of the drug for the treatment of endometriosis. The implant of the present invention may have only gestrinone in its constitution, but it is preferably formed by particles of gestrinone dispersed homogeneously in a bioerodible and bioabsorbable polymeric matrix. This polymeric matrix can be formed by a polymer or a mixture of polymers. The implants can have any size, shape or structure that facilitates their manufacture and insertion, however, to obtain a more constant and uniform release of the asset, it is necessary to use geometric shapes that maintain their surface area over time.

## Description

The present application for the patent of invention is aimed at the medical sector and comprises a long-lasting resorbable subcutaneous implant with controlled release of pharmacologically active substance pre-concentrated in polymer for the treatment of endometriosis.

### BACKGROUND OF THE INVENTION

Gestrinone is an anabolic androgenic steroid drug widely used for the treatment of endometriosis. Endometriosis is a disease that affects women of reproductive age and is characterized by the presence of ectopic endometrial tissue and/or stroma outside the uterine cavity. The treatment of endometriosis can be done through surgery to remove the excess tissue or with the use of synthetic steroid drugs, such as danazol, gestrinone, progestogens or (GnRHa) agonists.

In treatment with danazol, patients report unwanted side effects related to its androgenic action. In treatment with (GnRHa) agonists, the unwanted side effects reported are symptoms of perimenopause. In the treatment with gestrinone, side effects have low incidence and intensity, since it is possible to make the drug effective and long-lasting with low dosages. Thus, gestrinone has advantages over other forms of treatment of endometriosis.

The mechanism of action of gestrinone for the treatment of estrogen dependent pathologies comes from its combined action to inhibit the release of gonadotropins, androgenic, anti-estrogenic and anti-progestogenic properties. Gestrinone acts on the hypothalamus-pituitary system by suppressing the release of luteinizing hormone (LH) and stimulating follicle (FSH), reducing estrogen synthesis. It also reduces the size of fragments of endometrial tissues, leading to its regression.

Unlike other drugs used to treat endometriosis, which require high dosages to show regression of excess endometrial tissues, gestrinone is effective at low dosages and does not require daily administration. The presentations available for the use of gestrinone are oral, vaginal or parenteral. In the oral treatment the recommended dose is 2.5 mg, twice a week. In more severe cases of the disease, the dose can be increased to 2.5 mg three times a week, and this drug is well tolerated by the body in doses of up to 5 mg three times a week.

The vaginal route can be used as an alternative to patients who have gastric intolerance or who suffer from many side effects of oral treatment. The drug is absorbed in the vaginal canal without first-pass metabolism, consequently having less liver toxicity, reducing the intensity of side effects. The dose used is the same as the oral route and the blood levels of the drug through the vaginal route are lower than those observed in the oral treatment.

The parental route is used from the incision of a needle hypodermic in the arm of patients and insertion of silastic implants. They must be removed at the end of the treatment. The recommended dosage is 3 to 5 implants with 30 to 40 mg of gestrinone each. The treatment lasts up to nine months. Unwanted side effects such as acne and seborrhea occur with less incidence in the parental route compared to the oral and vaginal routes the form that has the least unwanted effects.

Traditional routes of administration have several disadvantages compared to the innovation proposed in this document. The oral and vaginal route, for example, are forms of treatment totally dependent on the patient, and it is necessary to count on their adherence and commitment to use the recommended doses at the intervals indicated by the doctor. Adherence to treatment is known to be one of the main factors related to the success or failure of a therapeutic approach. Chronic diseases such as endometriosis, cause the patient to have to take at least one medication indefinitely to keep the disease under control. The combination of long-term treatment and the absence of symptoms after medication can affect the correct use of the medication by the patient. Chronic disease patients are more likely to decrease their adherence to the drug over time.

Another disadvantage in the oral and vaginal use of gestrinone is the ease in making improper use of the prescribed medication, increasing the risks of underdoses or supra physiological doses, leading patients to a possible recurrence of symptoms, compromising their general condition in the treatment. [10] The most effective way to increase adherence to treatment by part of the patients is the simplification of medication dosages. For many chronic diseases, the development of drugs with prolonged release has made it possible to simplify dosages.

In this way, it is possible to find a third option known as gestrinone implants or bioabsorbable pellets for the treatment of endometriosis. Such implants have sustained release of the drug in order to treat and avoid adverse symptoms of the disease.

The terms "implant" or "pellet" refer to this dosage form already consolidated in the official collections of standards for medicines and pharmaceutical substances. They are characterized by being solid and sterile preparations of adequate size and shape for parenteral implantation and release of the active substance(s) over an extended period of time.

The terms "continuous release", "controlled release" or "sustained release" refer to the form of drug release through the implant, which occurs continuously and gradually over an extended period of time and does not result in an immediate and concentrated release of the drug in the body.

Referring to patent documents for resorbable implants, US4957119 *(Contraceptive implant)* mentions an implant of polymeric material that can release a contraceptive agent for a relatively long time when adjusted subcutaneously or locally. The implant comprises an ethylene/vinyl acetate copolymer core material that functions as a matrix for a contraceptive substance, an ethylene/vinyl acetate membrane surrounding the core material and a contact layer at the interface of the core and membrane material that prevents separation of the core material from the membrane. The implant of the invention is made by a co-extrusion process.

Biodegradable polymers or bioerodible polymers refer to a polymer that degrades in vivo and that its erosion over time occurs concomitantly with and/or subsequently the release of the therapeutic agent. A biodegradable polymer can be a homopolymer, copolymer or a polymer compressing more than two polymer units. In some cases, a biodegradable polymer may include a mixture of two or more homopolymers or copolymers.

The second patent document US9980850 (Bioerodible *contraceptive implant and methods of use thereof*) describes a bioerodible contraceptive implant and methods of use in the form of a controlled release bioerodible granule for subdermal implantation. The bioerodible sediment provides the sustained release of a contraceptive agent for an extended period. Bioerosion products are water-soluble, bioresorbed or both, avoiding the need for surgical removal of the implant.

Biodegradable implants or bioerodible implants can be understood as implants that have some mechanism that gradually reduces their mass over an extended period of time of release. The forces involved in this mass reduction may be cell interaction or shear forces on the implant surface. In addition, erosion and gradual dissolution of its components are possible. The terms also refer to the total degradation and absorption by the organism that occurs at the place where the implants were applied, excluding the need to remove the implants at the end of the treatment.

Although silastic implants improve therapeutic adherence, as treatment does not depend on the patient to take the medication, they have some disadvantages. The silastic implant has a non-degradable external membrane, making it necessary to remove this implant at the end of the treatment through minor surgery. This setback means that patients often do not opt for this type of treatment. In addition, for the silastic implant to be removed, it is necessary that the professional responsible for carrying out the insertion has done it correctly because if the implant is placed in a very deep layer, the removal process can be harmful to the tissues and painful for the patient, causing local inflammatory reactions. Another disadvantage is that there is the possibility of rupture of the membrane that lines the silastic implant, causing the drug to be dumped into the body which can cause unwanted side effects due to the sudden increase of the drug in the body. Finally, another disadvantage is related to the geometric shapes of these implants, which do not make treatment regular.

Observing the pre-existing deficiencies and problems in conventional standards aimed at treating endometriosis, the present invention aims to reduce pain and inflammation through a resorbable subcutaneous implant, with controlled release of the active substance, capable of releasing the drug directly into the bloodstream in order to prevent hepatic metabolism, thus producing the minimization of unwanted side effects and a better life quality for patients. Another advantage of the present invention is the elimination of residues in the tissues after the end of the treatment, which rules out the need for surgery to remove the implant.

### DESCRIPTION OF THE DRAWINGS

For a better understanding of the present invention, the following figures are attached:
Figure 1 - shows the chemical structure of the compound gestrinone
Figure 2 - shows dimensional designs of gestrinone implants
Figure 3 - shows dimensional designs of gestrinone implants
Figure 4 - Shows a graph illustrating the profile of the release rate of a bioabsorbable gestrinone implant in phosphate-saline buffer medium
Figure 5 - Shows a graph depicting the representation of the mathematical adjustment of the drug release kinetics, described by the Higuchi model (pseudo-first order).

### Detailed description of the invention

The present invention is a biodegradable implant of gestrinone in a polymeric matrix. The implant is inserted subdermally and has a continuous release of the asset for an extended period of time. This release aims to guarantee an efficient, constant and prolonged serum level of the drug for the treatment of endometriosis.

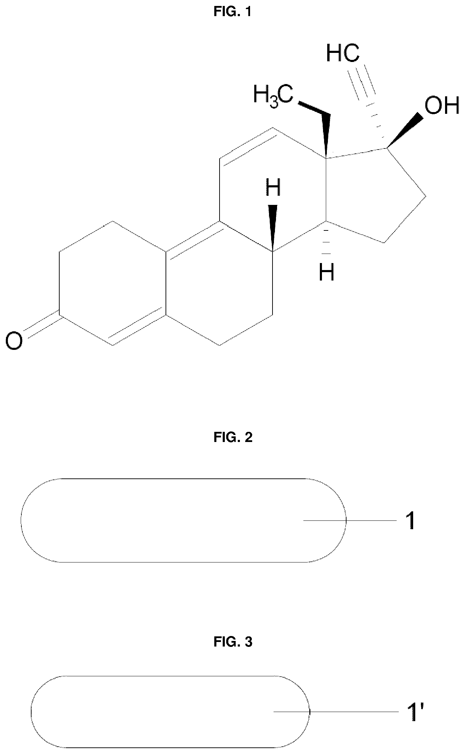

The "active substance", "active" or "drug" refers to the synthetic steroid gestrinone, which has the chemical structure shown in figure 1.

The implant of the present invention may have only gestrinone in its constitution, but it is preferably formed by particles of gestrinone dispersed homogeneously in a bioerodible and bioabsorbable polymeric matrix. This polymeric matrix can be formed by a polymer or a mixture of polymers. The amount of gestrinone present in the implant can vary from 5 to 200 mg per implant and its composition has from 1 to 20% of biodegradable polymer in proportion to the weight. Preferably it should have 20 to 110 mg and 2 to 10% of biodegradable polymer in proportion to the weight.

The biodegradable polymer used can be: Poly (D-lactic acid), Poly (L-lactic acid), Poly (racemic lactic acid), Poly (glycolic acid), Poly (caprolactone), methylcellulose, ethylcellulose, hydroxypropylcellulose (HPC) hydroxypropylmethylcellulose (HPMC), polyvinylpyrrolidone (PVP), poly (vinyl alcohol) (PVA), poly (ethylene oxide) (PEO), polyethylene glycol, starch, natural and synthetic gum and wax.

The implants can have any size, shape or structure that facilitates their manufacture and insertion, however, to obtain a more constant and uniform release of the asset, it is necessary to use geometric shapes that maintain their surface area over time.

Thus, the implant developed and demonstrated in the present application adopts the cylindrical pattern, in the shape of a rod, provided with straight or rounded tips, with length between 2 to 20 mm and the diameter from 1 to 6 mm. Schematic drawings of some examples of implant dimensions (1) and (1') are shown in figures 2 and 3.

The implant can have a polymeric coating membrane, with a thickness between 0.1 to 0.7 mm. The polymer used for the coating must be bioabsorbable and allow the passage of the asset. The coating of the implant is preferably done by immersing the implant in a polymeric solution. The coating can cover the total surface of the implant including the edges, only its longitudinal surface with the edges without coating or coated only on the edges of the implant without covering its length. The polymers that can be used for the coating are: copolymers of methyl vinyl siloxane, dimethylpolysiloxane, polylactic-glycolic acid (PLGA) and copolymers of D, L-lactic acid.

The manufacture of the gestrinone implant can be made from the addition of the drug in the solution of the chosen polymeric matrix in a proportion of 1 to 20% of biodegradable polymer in relation to the weight of the drug, with the formation of a homogeneous mixture. If the system solvent is not also a drug solvent, it will be dispersed in the form of particles or solution, and a *mixer* can be used to make the solution homogeneous. This solution is then dried and shaped into the desired format.

Another possible way of manufacturing the gestrinone implant is from the mixture of the drug and the polymeric matrix chosen in its dry, powdered forms. The drug and the polymeric matrix are added in a proportion of 1 to 20% of biodegradable polymer in relation to the weight of the drug in a suitable container and the mixture is homogenized.

The mixture of active ingredients to manufacture the implant can be molded from pressure or heat, so as not to compromise the effectiveness of the drug or to degrade the polymeric material. The technical options for implant impression can be: injection molding, hot molding, compression molding or extrusion molding.

For the present invention, the technique chosen was compression molding. In this technique, the mixing of the active ingredients, in powder form, is added to a mold and there is the application of mechanical force under the mixture, generating the compression of the particles and consequently the molding of the implant. Then there is the filling and sterilization of the gestrinone implant. Its sterilization can be done by heat or by gamma rays. Sterilization is preferably done by heat, in an oven at 90 °C, for 1 hour.

The proposed implant has a duration of approximately 4.5 to 6 months, this time being the period proposed between implant insertions. The complete treatment lasts according to the remission of the endometriosis markers. The therapeutic window for gestrinone in the scientific literature orally is 2.5 mg - twice a week, up to 5 mg - three times a week. In total, the amount of active that can be administered to the patient varies from 20 to 60 mg monthly. Since the treatment with the proposed implant has a minimum duration of approximately 4.5 months, the dose used, drawing a parallel with the dose used orally, can vary from 90 to 270 mg. The maximum dose, for example, is equivalent to approximately eight 35 mg gestrinone implants. However, the number of implants used in the treatment must be defined based on the assessment of the patient's clinical condition and analyzing the remission of the symptoms of endometriosis with the dose used. If necessary, the dose can also be adjusted by inserting additional implants. If rejection or any adverse reaction occurs after the implant is inserted, it can be removed within the first days of treatment. It should be noted that after a certain period of time this procedure will not be possible due to the biochemical decomposition of the polymer in vivo.

The use of the implant proposed here is safe and effective in the treatment of endometriosis, considering that the therapy does not depend on the patient's will or discipline for the action of the medication, thus ensuring the maintenance of the dosage and regularity of the treatment. It should be noted that patients with chronic pathologies often discontinue treatment when they notice the reversal of symptoms or when they feel some discomfort due to side effects or adverse reactions, becoming more susceptible to an aggravation of their clinical condition. Therefore, the use of these implants in the therapeutic approach prevents discontinuation and guarantees the appropriate treatment, as well as its effectiveness.

In addition, the invention provides endometriosis patients with constant and stable doses of continuous release of the active ingredient. This release occurs for an estimated period of 4.5 months and has a release of 0.09 mg of the drug per day, but not linearly over time, but proportional to the square root of the application time. The traditional form of oral treatment releases approximately 0.7 to 2.0 mg depending on the dosage used. The release through the implant is much less, causing no toxicity peaks or underdoses, thus preventing fluctuations in the therapeutic window.

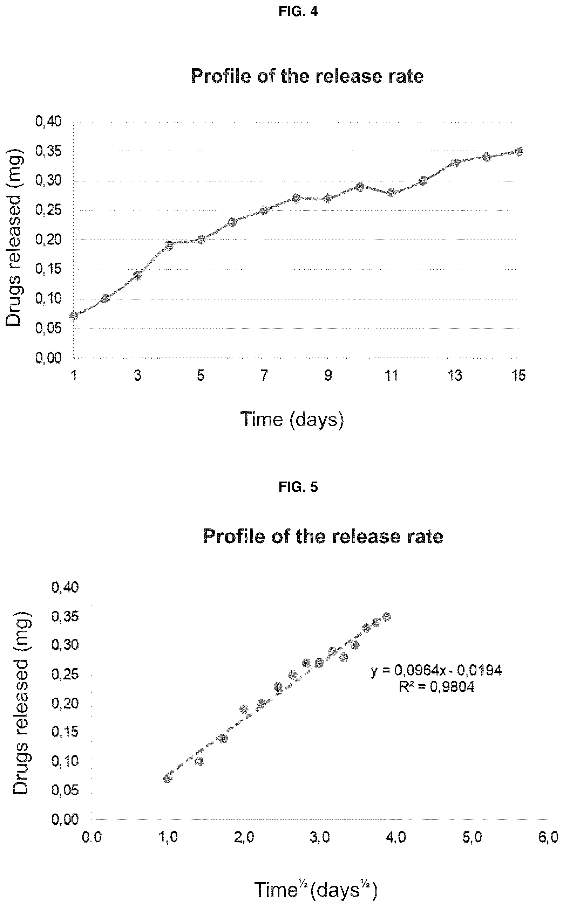

Figure 4 shows the profile of the rate of release of gestrinone over time from the bioabsorbable implant. This release rate profile was found through an in vitro test, which sought to find the in vitro-in vivo (IVIVC) correlation between the pharmaceutical form of the implant of gestrinone and a relevant in vivo response.

Figure 5 shows the mathematical adjustment of the release rate profile found in the IVIC dissolution test carried out from the bioabsorbable gestrinone implant. The release profile of figure 4 was adjusted according to some mathematical models and it was observed, from the analysis of the coefficient of determination (R²), that the mathematical model that best fitted the data was the Higuchi kinetic model, also called pseudo-first order. Thus, the bioabsorbable gestrinone implant in question has pseudo-first order release kinetics, in which the drug release is proportional to the square root of the application time, as mentioned earlier.

Another benefit of this invention is that the release of the drug through the implants occurs directly in the bloodstream, which makes its action much more efficient and prevents hepatic metabolization of the drug. Thus, the dose required for treatment can be reduced, minimizing unwanted side effects.

Another advantage of the invention is in the type and form in which the implant is presented, biodegradable and resorbable, and its removal is not necessary after the treatment period, since it does not leave residues in the tissues.

Research was conducted to assess the effectiveness of using the gestrinone implants described here for the treatment of endometriosis. About 1,000 patients with different degrees of the disease were followed up in clinical practice and the dose used was 1 to 3 implants. In patients with more severe endometriosis, with indication for surgery, the treatment with the implant resulted in a reduction in pain and inflammation, serving as an auxiliary treatment to the surgical intervention. In patients with superficial endometriosis, without indication for surgery, in which cells similar to the endometrium have extensive peritoneal coverage, but not deep, that is, they do not advance more than 5 mm, the treatment with gestrinone shows complete remission of pain symptoms and inflammation in the vast majority of cases. In these patients, treatment can be used only.

In addition to reducing pain and inflammation, it has been observed in practice that gestrinone improves the quality of life of patients as a whole, improving mood, increasing libido, improving reasoning and decisionmaking capacity and increasing lean mass. This is due to one of the mechanisms of action of gestrinone in the body, in which there is a reduction in the carrier protein SHBG in the patient, resulting in an increase in free circulating testosterone. This increase in free circulating testosterone generates all the benefits described above.

Another use of the described implant is to prevent and treat estrogen and progestogen dependent diseases, such as meningiomas and tumors, whether benign or malignant. Gestrinone, being derived from didrotestosterone, decreases aromatization to estrone, leading to an improvement in tumors.

## Claims

1. Long-term re-absorbable subcutaneous implant with controled pre-concentrated pharmacologically active polymer substance release for treating endometriosis", characterized for there is in the constitution of the biodegradable implant 5 to 200 mg of gestrinone in particles homogeneously dispersed in a polymeric matrix bioerodible and the bioabsorbable composition, being the bio-absorbable matrix, and the bio-absorbable matrix, from 1 to 20% biodegradable polymer in proportion to weight;

2. Long-term re-absorbable subcutaneous implant with controled pre-concentrated pharmacologically active polymer substance release for treating endometriosis,
according to claim 1, **characterized by** the fact that the biodegradable polymer is poli (d-lactic acid), poli (I-lactic acid), poli (racemic lactic acid), poly (glycolic acid), poly (caprolactone), methylcellulose, ethylcellulose, hydroxypropylcellulose (hpc) hydroxypropylmethylcellulose (hpmc), polyvinylpyrrolidone (pvp), poly (vinyl alcohol) (pva), poly (ethylene oxide) (peo), polyethylene glycol, starch, natural gum, synthetic gum and wax;

3. Long-term re-absorbable subcutaneous implant with controled pre-concentrated pharmacologically active polymer substance release for treating endometriosis, according to claim 1, **characterized by** the amount of gestrinone present in the implant varying from 20 to 110 mg;

4. Long-term reabsorbible subcutaneous implant with controled pre-concentrated pharmacologically active polymer substance release for treating endometriosis, according to claims 1, 2 and 3, **characterized by** the geometric shape of the implant (1) and (1') adopt the cylindrical, rod-shaped pattern, provided with straight or rounded tips, with a length between 2 to 20 mm and a diameter of 1 to 6 mm.

5. Long-term reabsorbible subcutaneous implant with controled pre-concentrated pharmacologically active polymer substance release for treating endometriosis, according to claims 1 to 4, **characterized by** the fact that the implant has a coating formed by a bioabsorbable polymeric membrane, with thickness between 0.1 to 0,7 mm. Said membrane coming from copolymers of methyl vinyl siloxane, dimethylpolysiloxane, polylactic acid glycolic (PLGA) and copolymers of D, L-lactic acid;

6. Long-term reabsorbible subcutaneous implant with controled pre-concentrated pharmacologically active polymer substance release for treating endometriosis, according to claims 1 to 5, **characterized by** the impression of the implant being performed by compression, where the mixing of the assets, in powder form, is added to a mold and mechanical force is applied to the mixture, generating the compression of the particles and consequently the molding of the implant, with the filling and sterilization of the implant gestrinone by heat, in an oven at 90 °C, for 1 hour, or by gamma rays;

7. Long-term reabsorbible subcutaneous implant with controled pre-concentrated pharmacologically active polymer substance release for treating endometriosis, according to claims 1 to 6, **characterized by** the release of the drug (gestrinone) in the amount of 0.09 mg per day, proportional to the square root of application time.
